# DEMANDE DE BREVET EUROPEEN

(11) **EP 1 055 409 A1**
(43) Date de publication de la demande: **29.11.2000**
(21) Numéro de dépôt: 00401360.3
(22) Date de dépôt: 18.05.2000
(51) Int. Cl.: A61K 7/13

(54) **Compositioin de teinture d'oxydation des fibres kératiniques et procédé de teinture mettant en oeuvre cette composition**

(30) Priorité: 28.05.1999 FR 9906799
(71) Demandeur: L'OREAL, 75008 Paris (FR)
(72) Inventeur: Lang, Gérard, 95390 Saint Prix (FR); Lagrange, Alain, 77700 Coupvray (FR)
(74) Mandataire: Miszputen, Laurent

(57) **Abrégé**

L'invention a pour objet une composition prête à l'emploi pour la teinture d'oxydation des fibres kératiniques, et en particulier des fibres kératiniques humaines telles que les cheveux comprenant, dans un milieu approprié pour la teinture, au moins une base d'oxydation choisie parmi les paraphénylènediamines, les bases doubles, les para-aminophénols et les bases hétérocycliques, au moins une enzyme de type oxydo-réductase à 2 électrons en présence d'au moins un donneur pour ladite enzyme, et au moins un médiateur enzymatique; ainsi que le procédé de teinture d'oxydation mettant en oeuvre cette composition.

## Description

L'invention a pour objet une composition prête à l'emploi pour la teinture d'oxydation des fibres kératiniques, et en particulier des fibres kératiniques humaines telles que les cheveux comprenant, dans un milieu approprié pour la teinture, au moins une base d'oxydation choisie parmi les paraphénylènediamines, les bases doubles, les para-aminophénols et les bases hétérocycliques, au moins une enzyme de type oxydo-réductase à 2 électrons en présence d'au moins un donneur pour ladite enzyme, et au moins un médiateur enzymatique ; ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les méta-aminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présentent pour inconvénient d'entraîner une dégradation non négligeable des fibres, ainsi qu'une décoloration importante des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé de teindre les fibres kératiniques, notamment dans la demande de brevet EP-A-0 310 675, avec des compositions comprenant une base d'oxydation et éventuellement un coupleur, en association avec des enzymes telles que la pyranose-oxydase, la glucose-oxydase ou bien l'uricase, en présence d'un donneur pour lesdites enzymes. Ces procédés de teinture, bien qu'étant mis en oeuvre dans des conditions n'entraînant pas une dégradation des fibres kératiniques comparable à celle engendrée par les teintures réalisées en présence de peroxyde d'hydrogène, conduisent à des colorations ne donnant pas entière satisfaction notamment du point de vue de leur intensité et de leur résistance vis à vis des diverses agressions que peuvent subir les cheveux.

Il a déjà également été proposé, notamment dans la demande de brevet WO 98/40471, de teindre les fibres kératiniques avec des compositions comprenant notamment un précurseur de colorant d'oxydation, une enzyme de type laccase (oxydo-réductase à 4 électrons) et un médiateur enzymatique. Cependant les colorations obtenues en mettant en oeuvre ces compositions ne donnent pas toujours satisfaction en ce qui concerne l'intensité et la résistance des colorations obtenues vis à vis des diverses agressions que peuvent subir les cheveux.

D'autre part, l'utilisation de médiateur enzymatique est bien connue dans l'industrie du papier pour le blanchiment et la délignification de la pulpe de papier , voir en particulier H. P. Call et al., Journal of Biotechnology 53 (1997) 163-202.

Or, la demanderesse vient maintenant de découvrir qu'il est possible d'obtenir de nouvelles teintures, capables de conduire à des colorations puissantes sans engendrer de dégradation significative des fibres kératiniques, peu sélectives et résistant bien aux diverses agressions que peuvent subir les fibres, en associant au moins une base d'oxydation choisie parmi les paraphénylènediamines, les para-aminophénols, les bases doubles, et les bases hétérocycliques, au moins une enzyme de type oxydo-réductase à 2 électrons en présence d'au moins un donneur pour ladite enzyme, et au moins un médiateur enzymatique sélectionné.

Cette découverte est à la base de la présente invention.

L'invention a donc pour premier objet une composition prête à l'emploi, pour la teinture d'oxydation des fibres kératiniques et en particulier des fibres kératiniques humaines telles que les cheveux, caractérisée par le fait qu'elle comprend, dans un milieu approprié pour la teinture :
- au moins une base d'oxydation choisie parmi :
   i) les paraphénylènediamines de formule (I) suivante, et leurs sels d'addition avec un acide : 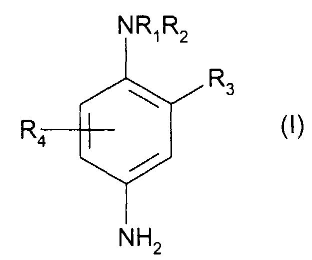 dans laquelle :
      - R₁ et R₂, identiques ou différents, représentent un atome d'hydrogène, un radical alkyle en C₁-C₄, ou monohydroxyalkyle en C₁-C₄ ;
      - R₃ représente un atome d'hydrogène ou d'halogène, un radical alkyle en C₁-C₄, ou monohydroxyalkyle en C₁-C₄ ;
      - R₄ représente représentent un atome d'hydrogène ou un radical alkyle en C₁-C₄ ;
   ii) les bases doubles ;
   iii) les para-aminophénols de formule (II) suivante et leurs sels d'addition avec un acide : 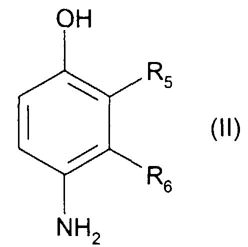 dans laquelle :
      - R₅ et R₆, identiques ou différents, représentent un atome d'hydrogène ou d'halogène, un radical alkyle en C₁-C₄, monohydroxyalkyle en C₁-C₄, alcoxy(C₁-C₄)alkyle en C₁-C₄, aminoalkyle en C₁-C₄, monohydroxyalkyle(C₁-C₄)aminoalkyle en C₁-C₄,
      étant entendu qu'au moins un des radicaux R₅ et R₆ représente un atome d'hydrogène ;
   iv) les bases hétérocycliques ;
      - au moins une enzyme de type oxydo-réductase à 2 électrons ;
      - au moins un donneur pour ladite enzyme ; et
      - au moins un médiateur enzymatique capable d'augmenter l'activité enzymatique de ladite oxydoréductase à 2 électrons.

La composition tinctoriale prête à l'emploi conforme à l'invention conduit rapidement à des colorations puissantes présentant une faible sélectivité et d'excellentes propriétés de résistances à la fois vis à vis des agents atmosphériques tels que la lumière et les intempéries et vis à vis de la transpiration et des différents traitements que peuvent subir les cheveux.

L'invention a également pour objet un procédé de teinture d'oxydation des fibres kératiniques mettant en oeuvre cette composition tinctoriale prête à l'emploi.

La ou les oxydo-réductases à 2 électrons utilisées dans la composition tinctoriale prête à l'emploi conforme à l'invention peuvent notamment être choisies parmi les pyranose oxydases, les glucose oxydases, les glycérol oxydases, les lactates oxydases, les pyruvate oxydases, les uricases, les choline oxydases, les sarcosine oxydase, et les bilirubine oxydases.

Selon l'invention, l'oxydo-réductase à 2 électrons est de préférence choisie parmi les uricases d'origine animale, microbiologique ou biotechnologique.

A titre d'exemple, on peut notamment citer l'uricase extraite de foie de sanglier, l'uricase d'Arthrobacter globiformis, ainsi que l'uricase d'Aspergillus flavus.

La ou les oxydo-réductases à 2 électrons peuvent être utilisées sous forme cristalline pure ou sous une forme diluée dans un diluant inerte pour ladite oxydo-réductase à 2 électrons.

La ou les oxydo-réductases à 2 électrons conformes à l'invention représentent de préférence de 0,01 à 20 % en poids environ du poids total de la composition tinctoriale prête à l'emploi, et encore plus préférentiellement de 0,1 à 5 % en poids environ de ce poids.

Selon l'invention, on entend par donneur, les différents substrats participant au fonctionnement de ladite ou desdites oxydo-réductases à 2 électrons.

La nature du donneur (ou substrat) pour ladite enzyme varie en fonction de la nature de l'oxydo-réductase à 2 électrons qui est utilisée. Par exemple, à titre de donneur pour les pyranose oxydases, on peut citer le D-glucose, le L-sorbose et le D-xylose ; à titre de donneur pour les glucose oxydases, on peut citer le D-glucose, à titre de donneur pour les glycérol oxydases, on peut citer le glycérol et la dihydroxyacétone ; à titre de donneur pour les lactate oxydases, on peut citer l'acide lactique et ses sels ; à titre de donneur pour les pyruvate oxydases, on peut citer l'acide pyruvique et ses sels ; à titre de donneur pour les uricases, on peut citer l'acide urique et ses sels ; à titre de donneur pour les choline oxydases, on peut citer la choline et ses sels d'addition avec un acide comme le chlorhydrate de choline, et la bétaïne aldéhyde ; à titre de donneur pour les sarcosine oxydases, on peut citer la sarcosine, la N-méthyl-L-leucine, la N-méthyl-DL-alanine, et la N-méthyl-DL-valine ; et enfin, à titre de donneur pour les bilirubine oxydases, on peut citer la bilirubine.

Le ou les donneurs (ou substrats) utilisés conformément à l'invention représentent de préférence de 0,01 à 20 % en poids environ du poids total de la composition tinctoriale prête à l'emploi conforme à l'invention et encore plus préférentiellement de 0,1 à 5 % en environ de ce poids.

Le ou les médiateurs enzymatiques utilisés dans la composition prête à l'emploi conforme à l'invention permettent d'augmenter l'activité enzymatique de l'oxydoréductase utilisée et peuvent par exemple être choisis parmi les composés de formule (III) suivante, et leurs possibles formes tautomères : 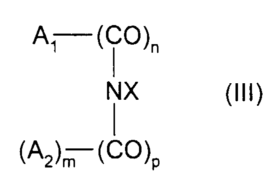 dans laquelle :
- A₁ et A₂, identiques ou différents, représentent :
   a) un radical aliphatique saturé ou insaturé, linéaire ou ramifié, comportant de 1 à 30 atomes de carbone, ledit radical aliphatique pouvant ou non être substitué par un ou plusieurs radicaux hydroxyle, halogène, sulfo, carboxy, nitro ou phényle ;
   b) un radical hétérocyclique comprenant de 1 à 4 hétéroatomes et de 5 à 10 chaînons pouvant ou non être substitué par un ou plusieurs radicaux alkyle en C₁-C₄, halogène, phényle, hydroxyle, ou aralkyle en C₇-C₁₀ ;
   c) un radical aromatique comprenant de 6 à 10 chaînons, ledit radical aromatique pouvant ou non être substitué par un ou plusieurs radicaux alkyle en C₁-C₄, halogène, sulfo, carboxy, nitro, hydroxyle, ou nitroso ;
   l'atome d'azote du groupement NX pouvant former avec les groupements A₁-(CO)ₙ et A₂-(CO)ₚ un hétérocycle comprenant de 5 à 18 chaînons, ledit hétérocycle pouvant ou non être substitué par un ou plusieurs radicaux alkyle en C₁-C₄, hydroxyle, phényle, halogène, sulfo, carboxy ou nitro ;
- X représente un groupement -OH, =O, =S, →O ou →S ;
- m, n et p, indépendamment les uns des autres, sont des nombres entiers égaux à 0 ou 1.

Parmi les médiateurs enzymatiques de formule (III) ci-dessus, on peut notamment citer l'hydroxylamine, la N,N-dipropyl hydroxylamine, la N,N-diisopropyl hydroxylamine, la phényl hydroxylamine, la N-acétyl hydroxylamine, le 1-phényl-1H-1,2,3-triazole-1-oxyde, le 2,4,5-triphényl-2H-1,2,3-triazol-1-oxyde, le 1-hydroxy benzotriazole, l'acide 1-hydroxy benzotriazole sulfonique, le 1-hydroxy benzimidazole, le N-hydroxy phtalimide, le N-hydroxy succinimide, la quinoline N-oxyde, l'isoquinoline N-oxyde, la 1-hydroxy pipéridine, l'acide violurique, la 4-hydroxy 3-nitroso coumarine, l'acide 1,3-diméthyl 5-nitroso barbiturique, le 1-nitroso 2-naphtol, l'acide 2-nitroso 1-naphtol 4-sulfonique, le 2-nitroso 1-naphtol, l'acide 1-nitroso 2-naphtol 3,6-disulfonique, et le 2,4-dinitroso 1,3-dihydroxy benzène.

Le ou les médiateurs enzymatiques utilisés dans la composition prête à l'emploi conforme à l'invention peuvent également être choisis parmi l'acide syringique et ses esters ; l'acétosyringone ; le syringaldéhyde ; l'acide parahydroxycinnamique ; la vanilline ; la 7-hydroxycoumarine ; le 2,4-dichloro phénol ; le parahydroxybenzènesulfonate ; le 2,2'-azino-bis-(3-éthylbenzothiazoline-6-sulfonate) ; les phénothiazines telles que la 10-méthyl phénothiazine ; les benzidines telles que la 3,3'-diméthyl benzidine ; les dérivés aminés de l'acide 2-naphtalène sulfonique ; la L-tyrosine ; l'acide férulique ; l'acide caféique, l'acide chlorogénique, et l'acide sinapique.

Le ou les médiateurs enzymatiques utilisés dans la composition tinctoriale prête à l'emploi conforme à l'invention représentent de préférence de 0,0001 à 5 % en poids environ par rapport au poids total de la composition tinctoriale prête à l'emploi et encore plus préférentiellement de 0,005 à 0,5 % en poids environ de ce poids.

Parmi les paraphénylènediamines de formule (I) décrite ci-dessus et utilisables à titre de base d'oxydation dans la composition tinctoriale prête à l'emploi conforme à l'invention, on peut notamment citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxy-éthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, et leurs sels d'addition avec un acide.

Parmi les bases doubles utilisables à titre de base d'oxydation dans la composition tinctoriale prête à l'emploi conforme à l'invention, on peut notamment citer les composés comportant au moins deux noyaux aromatiques sur lesquels sont portés plusieurs groupements amino et/ou hydroxyle.

Parmi lesdites bases doubles on peut plus particulièrement citer les composés de formule (IV) suivante, et leurs sels d'addition avec un acide : 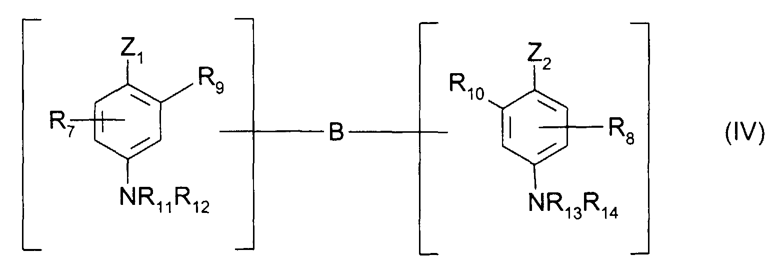 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison B représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₇ et R₈ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₉, R₁₀, R₁₁, R₁₂, R₁₃ et R₁₄, identiques ou différents, représentent un atome d'hydrogène, un bras de liaison B ou un radical alkyle en C₁-C₄ ;
étant entendu que les composés de formule (IV) ne comportent qu'un seul bras de liaison B par molécule.

Parmi les groupements azotés de la formule (IV) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formule (IV) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (IV), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols de formule (II) décrite ci-dessus et pouvant être utilisés à titre de base d'oxydation dans la composition tinctoriale prête à l'emploi conforme à l'invention, on peut plus particulièrement citer le paraaminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les bases hétérocycliques utilisables à titre de base d'oxydation dans la composition tinctoriale prête à l'emploi conforme à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine; le 3-amino pyrazolo-[1,5-a]-pyrimidin-7-ol ; le 3-amino pyrazolo-[1,5-a]-pyrimidin-5-ol; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Selon l'invention, la ou les bases d'oxydation représentent de préférence de 0,0005 à 12 % en poids environ du poids total de la composition tinctoriale prête à l'emploi, et encore plus préférentiellement de 0,005 à 6 % en poids environ de ce poids.

Selon une forme de réalisation préférée, la composition tinctoriale prête à l'emploi conforme à l'invention peut en outre renfermer un ou plusieurs coupleurs et/ou un ou plusieurs colorants directs notamment pour modifier les nuances ou les enrichir en reflets.

Parmi les coupleurs pouvant être présents à titre additionnel dans la composition tinctoriale prête à l'emploi conforme à l'invention, on peut notamment citer les méta-aminophénols, les méta-phénylènediamines, les métadiphénols, les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs additionnels représentent de préférence de 0,0001 à 10 % en poids environ du poids total de la composition tinctoriale prête à l'emploi et encore plus préférentiellement de 0,005 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale prête à l'emploi, et encore plus préférentiellement entre 5 et 30 % en poids environ.

Le pH de la composition prête à l'emploi conforme à l'invention est choisi de telle manière que l'activité enzymatique de l'oxydo-réductase à 2 électrons soit suffisante. Il est généralement compris entre 3 et 11 environ, et de préférence entre 4 et 9 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino 1-propanol ainsi que leurs dérivés, les hydroxydes de sodium ou de potassium et les composés de formule (V) suivante : 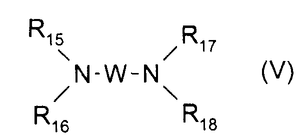 dans laquelle W est un reste propylène éventuellement substitué par un groupement hydroxyle ou un radical alkyle en C₁-C₄ ; R₁₅, R₁₆, R₁₇ et R₁₈, identiques ou différents, représentent un atome d'hydrogène, un radical alkyle en C₁-C₄ ou hydroxyalkyle en C₁-C₄.

La composition tinctoriale prête à l'emploi conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des enzymes différentes des oxydo-réductases à 2 électrons utilisées conformément à l'invention telles que par exemples des peroxydases, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains. Dans ce cas, les colorants d'oxydation et la ou les oxydo-réductases à 2 électrons sont présents au sein de la même composition prête à l'emploi, et par conséquent ladite composition doit être exempte d'oxygène gazeux, de manière à éviter toute oxydation prématurée du ou des colorants d'oxydation.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres, à une température d'application comprise entre la température ambiante et 80°C, au moins une composition tinctoriale prête à l'emploi telle que définie précédemment, pendant un temps suffisant pour développer la coloration désirée. De façon préférentielle, les fibres sont ensuite rincées, on éventuellement lavées au shampooing, puis séchées.

La température d'application est de préférence comprise entre la température ambiante et 60°C et encore plus préférentiellement entre 35°C et 50°C.

Le temps suffisant au développement de la coloration sur les fibres kératiniques est généralement compris entre 1 et 60 minutes et encore plus précisément entre 5 et 30 minutes.

Selon une forme de réalisation particulière de l'invention, le procédé comporte une étape préliminaire consistant à stocker sous forme séparée, d'une part, une composition (A) comprenant, dans un milieu approprié pour la teinture, au moins une base d'oxydation telle que définie précédemment, et d'autre part, une composition (B) renfermant, dans un milieu approprié pour la teinture, au moins une enzyme de type oxydo-réductase à 2 électrons en présence d'au moins un donneur pour ladite enzyme, et au moins un médiateur enzymatique capable d'augmenter l'activité enzymatique de ladite oxydoréductase à 2 électrons, puis à procéder à leur mélange au moment de l'emploi avant d'appliquer ce mélange sur les fibres kératiniques.

Un autre objet de l'invention est un dispositif de teinture à plusieurs compartiments ou "kit" de teinture ou tout autre système de conditionnement à plusieurs compartiments dont au moins un premier compartiment renferme la composition (A) telle que définie ci-dessus et au moins un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

L'exemple qui suit est destiné à illustrer l'invention sans pour autant en limiter la portée.

### EXEMPLE DE TEINTURE

On a préparé la composition tinctoriale prête à l'emploi suivante (teneurs en grammes) :

| **COMPOSITION** | **1** |
|---|---|
| Paraphénylènediamine (base d'oxydation de formule (I)) | 0,324 |
| 1,3-dihydroxy benzène (coupleur) | 0,33 |
| 1-hydroxy benzotriazole (médiateur enzymatique) | 0,1 |
| Uricase d'Arthrobacter globiformis à 20 Unités Internationales (U.I.) / mg, commercialisée par la société Sigma | 1,5 |
| Acide urique | 1,5 |
| Support de teinture (*) | (*) |
| Eau déminéralisée q.s.p. | 100 g |

| | |
|---|---|
| (*) : Support de teinture: - Hydroxyéthylcellulose vendue sous la dénomination commerciale NATROSOL 250 HHR ® par la société AQUALON 1,0 g - Ethanol à 96° 20 g - 2-méthyl 2-amino propanol-1 q.s. pH 9,5 | |

La composition tinctoriale prête à l'emploi décrite ci-dessus a été appliquée sur des mèches de cheveux gris naturels à 90 % de blancs pendant 30 minutes à une température de 30°C. Les cheveux ont ensuite été rincés, lavés avec un shampooing standard, puis séchés.

Les cheveux ont été teints dans une nuance châtain clair mat.

## Revendications

1. Composition prête à l'emploi, pour la teinture d'oxydation des fibres kératiniques et en particulier des fibres kératiniques humaines telles que les cheveux, caractérisée par le fait qu'elle comprend, dans un milieu approprié pour la teinture :
- au moins une base d'oxydation choisie parmi :
i) les paraphénylènediamines de formule (I) suivante, et leurs sels d'addition avec un acide : 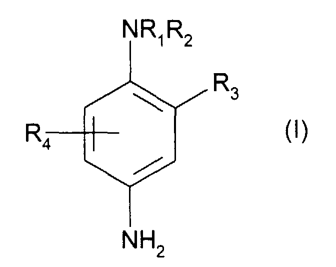 dans laquelle :
- R₁ et R₂, identiques ou différents, représentent un atome d'hydrogène, un radical alkyle en C₁-C₄, ou monohydroxyalkyle en C₁-C₄ ;
- R₃ représente un atome d'hydrogène ou d'halogène, un radical alkyle en C₁-C₄, ou monohydroxyalkyle en C₁-C₄ ;
- R₄ représente un atome d'hydrogène ou un radical alkyle en C₁-C₄ ;
ii) les bases doubles ;
iii) les para-aminophénols de formule (II) suivante et leurs sels d'addition avec un acide : 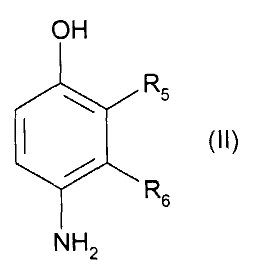 dans laquelle :
- R₅ et R₆, identiques ou différents, représentent un atome d'hydrogène ou d'halogène, un radical alkyle en C₁-C₄, monohydroxyalkyle en C₁-C₄, alcoxy(C₁-C₄)alkyle en C₁-C₄, aminoalkyle en C₁-C₄, monohydroxyalkyle(C₁-C₄)aminoalkyle en C₁-C₄,
étant entendu qu'au moins un des radicaux R₅ et R₆ représente un atome d'hydrogène ;
iv) les bases hétérocycliques ;
- au moins une enzyme de type oxydo-réductase à 2 électrons ;
- au moins un donneur pour ladite enzyme ; et
- au moins un médiateur enzymatique capable d'augmenter l'activité enzymatique de ladite oxydoréductase à 2 électrons.

2. Composition selon la revendication 1, caractérisée par le fait que les oxydo-réductases à 2 électrons sont choisies parmi les pyranose oxydases, les glucose oxydases, les glycérol oxydases, les lactates oxydases, les pyruvate oxydases, les uricases, les choline oxydases, les sarcosine oxydase, et les bilirubine oxydases.

3. Composition selon la revendication 2, caractérisée par le fait que les oxydo-réductases à 2 électrons sont choisies parmi les uricases d'origine animale, microbiologique ou biotechnologique.

4. Composition selon l'une quelconque des revendications précédentes, caractérisée par le fait que les oxydo-réductases à 2 électrons représentent de 0,01 à 20 % en poids du poids total de la composition tinctoriale prête à l'emploi.

5. Composition selon la revendication 4, caractérisée par le fait que les oxydo-réductases à 2 électrons représentent de 0,1 à 5 % en poids du poids total de la composition tinctoriale prête à l'emploi.

6. Composition selon l'une quelconque des revendications précédentes, caractérisée par le fait que le ou les donneurs représentent de 0,01 à 20 % en poids du poids total de la composition tinctoriale prête à l'emploi.

7. Composition selon la revendication 6, caractérisée par le fait que le ou les donneurs représentent de 0,1 à 5 % en poids du poids total de la composition tinctoriale prête à l'emploi.

8. Composition selon l'une quelconque des revendications précédentes, caractérisée par le fait que le ou les médiateurs enzymatiques sont choisis parmi les composés de formule (III) suivante, et leurs possibles formes tautomères : 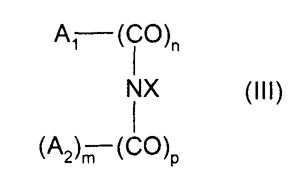 dans laquelle :
- A₁ et A₂, identiques ou différents, représentent :
a) un radical aliphatique saturé ou insaturé, linéaire ou ramifié, comportant de 1 à 30 atomes de carbone, ledit radical aliphatique pouvant ou non être substitué par un ou plusieurs radicaux hydroxyle, halogène, sulfo, carboxy, nitro ou phényle ;
b) un radical hétérocyclique comprenant de 1 à 4 hétéroatomes et de 5 à 10 chaînon, ledit radical hétérocyclique pouvant ou non être substitué par un ou plusieurs radicaux alkyle en C₁-C₄, halogène, phényle, hydroxyle, ou aralkyle en C₇-C₁₀ ;
c) un radical aromatique comprenant de 6 à 10 chaînons, ledit radical aromatique pouvant ou non être substitué par un ou plusieurs radicaux alkyle en C₁-C₄, halogène, sulfo, carboxy, nitro, hydroxyle, ou nitroso ;
l'atome d'azote du groupement NX pouvant former avec les groupements A₁-CO)ₙ et A₂-(CO)ₚ un hétérocycle comprenant de 5 à 18 chaînons, ledit hétérocycle pouvant ou non être substitué par un ou plusieurs radicaux alkyle en C₁-C₄, hydroxyle, phényle, halogène, sulfo, carboxy ou nitro ;
- X représente un groupement -OH, =O, =S, →O ou →S ;
- m, n et p, indépendamment les uns des autres, sont des nombres entiers égaux à 0 ou 1.

9. Composition selon la revendication 8, caractérisée par le fait que les médiateurs enzymatiques de formule (III) sont choisis parmi l'hydroxylamine, la N,N-dipropyl hydroxylamine, la N,N-diisopropyl hydroxylamine, la phényl hydroxylamine, la N-acétyl hydroxylamine, le 1-phényl-1H-1,2,3-triazole-1-oxyde, le 2,4,5-triphényl-2H-1,2,3-triazol-1-oxyde, le 1-hydroxy benzotriazole, l'acide 1-hydroxy benzotriazole sulfonique, le 1-hydroxy benzimidazole, le N-hydroxy phtalimide, le N-hydroxy succinimide, la quinoline N-oxyde, l'isoquinoline N-oxyde, la 1-hydroxy pipéridine, l'acide violurique, la 4-hydroxy 3-nitroso coumarine, l'acide 1,3-diméthyl 5-nitroso barbiturique, le 1-nitroso 2-naphtol, l'acide 2-nitroso 1-naphtol 4-sulfonique, le 2-nitroso 1-naphtol, l'acide 1-nitroso 2-naphtol 3,6-disulfonique, et le 2,4-dinitroso 1,3-dihydroxy benzène.

10. Composition selon l'une quelconque des revendications 1 à 7, caractérisée par le fait que le ou les médiateurs sont choisis parmi l'acide syringique et ses esters ; l'acétosyringone ; le syringaldéhyde ; l'acide parahydroxycinnamique ; la vanilline ; la 7-hydroxycoumarine ; le 2,4-dichloro phénol ; le parahydroxybenzènesulfonate ; le 2,2'-azino-bis-(3-éthylbenzothiazoline-6-sulfonate) ; les phénothiazines telles que la 10-méthyl phénothiazine ; les benzidines telles que la 3,3'-diméthyl benzidine ; les dérivés aminés de l'acide 2-naphtalène sulfonique ; la L-tyrosine ; l'acide férulique ; l'acide caféique, l'acide chlorogénique, et l'acide sinapique.

11. Composition selon l'une quelconque des revendications précédentes, caractérisée par le fait que le ou les médiateurs enzymatiques représentent de 0,0001 à 5 % en poids environ par rapport au poids total de la composition tinctoriale prête à l'emploi.

12. Composition selon la revendication 11, caractérisée par le fait que le ou les médiateurs enzymatiques représentent de 0,005 à 0,5 % en poids environ par rapport au poids total de la composition tinctoriale prête à l'emploi.

13. Composition selon l'une quelconque des revendications précédentes, caractérisée par le fait que les paraphénylènediamines de formule (I) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, et leurs sels d'addition avec un acide.

14. Composition selon l'une quelconque des revendications 1 à 12, caractérisée par le fait que les bases doubles sont choisies parmi les composés de formule (IV) suivante, et leurs sels d'addition avec un acide : 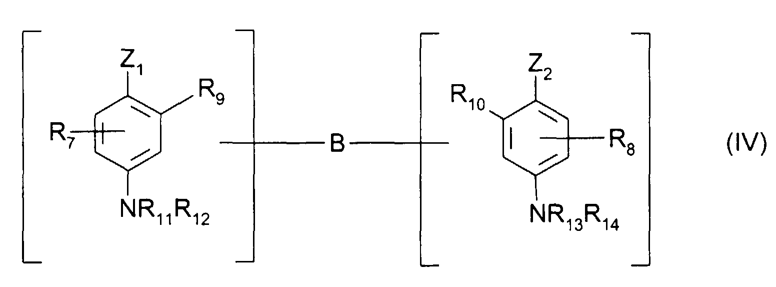 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison B représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₇ et R₈ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₉, R₁₀, R₁₁, R₁₂, R₁₃ et R₁₄, identiques ou différents, représentent un atome d'hydrogène, un bras de liaison B ou un radical alkyle en C₁-C₄ ;
étant entendu que les composés de formule (IV) ne comportent qu'un seul bras de liaison B par molécule.

15. Composition selon la revendication 14, caractérisée par le fait que les bases doubles de formule (IV) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

16. Composition selon l'une quelconque des revendications 1 à 12, caractérisée par le fait que les para-aminophénols de formule (II) sont choisis parmi le paraaminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

17. Composition selon l'une quelconque des revendications 1 à 12, caractérisée par le fait que les bases hétérocycliques sont choisies parmi les dérivés pyridiniques, les dérivés pyrimidiniques, les dérivés pyrazoliques, et leurs sels d'addition avec un acide.

18. Composition selon l'une quelconque des revendications précédentes, caractérisée par le fait que la ou les bases d'oxydation représentent de 0,0005 à 12 % en poids du poids total de la composition tinctoriale prête à l'emploi.

19. Composition selon la revendication 18, caractérisée par le fait que la ou les bases d'oxydation représentent de 0,005 à 6 % en poids du poids total de la composition tinctoriale prête à l'emploi.

20. Composition selon l'une quelconque des revendications précédentes, caractérisée par le fait qu'elle renferme un ou plusieurs coupleurs et/ou un ou plusieurs colorants directs.

21. Composition selon la revendication 20, caractérisée par le fait que les coupleurs sont choisis parmi les méta-aminophénols, les métaphénylènediamines, les métadiphénols, les coupleurs hétérocycliques, et leurs sels d'addition avec un acide.

22. Composition selon la revendication 21, caractérisée par le fait que les coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

23. Composition selon l'une quelconque des revendications précédentes, caractérisée par le fait que les sels d'addition avec un acide sont choisis parmi les chlorhydrates, les bromhydrates, les sulfates et les tartrates, les lactates et les acétates.

24. Composition selon l'une quelconque des revendications précédentes, caractérisée par le fait qu'elle présente un pH compris entre 4 et 9.

25. Procédé de teinture d'oxydation des fibres kératiniques et en particulier des fibres kératiniques humaines telles que les cheveux, caractérisé par le fait qu'on applique sur lesdites fibres, à une température d'application comprise entre la température ambiante et 80°C, au moins une composition tinctoriale prête à l'emploi telle que définie dans l'une quelconque des revendications précédentes, pendant un temps suffisant pour développer la coloration désirée.

26. Procédé selon la revendication 25, caractérisé par le fait que la température d'application est comprise entre 35°C et 50°C.

27. Procédé selon la revendication 25 ou 26; caractérisé par le fait que le temps suffisant au développement de la coloration est compris entre 1 et 60 minutes.

28. Procédé selon la revendication 27, caractérisé par le fait que le temps suffisant au développement de la coloration est compris entre 5 et 30 minutes.

29. Procédé selon l'une quelconque des revendications 25 à 28, caractérisé par le fait qu'il comporte une étape préliminaire consistant à stocker sous forme séparée, d'une part, une composition (A) comprenant, dans un milieu approprié pour la teinture, au moins une base d'oxydation telle que définie à l'une quelconque des revendications 1 et 13 à 19, et d'autre part, une composition (B) renfermant, dans un milieu approprié pour la teinture, au moins une enzyme de type oxydo-réductase à 2 électrons en présence d'au moins un donneur pour ladite enzyme, et au moins un médiateur enzymatique capable d'augmenter l'activité enzymatique de ladite oxydoréductase à 2 électrons, puis à procéder à leur mélange au moment de l'emploi avant d'appliquer ce mélange sur les fibres kératiniques.

30. Dispositif de teinture à plusieurs compartiments, caractérisé par le fait qu'il comporte au moins un premier compartiment renfermant la composition (A) telle que définie dans la revendication 29 et au moins un second compartiment renfermant la composition (B) telle que définie dans la revendication 29.
